# EUROPÄISCHE PATENTSCHRIFT

(11) **EP 1 384 069 B1**
(45) Veröffentlichungstag und Bekanntmachung des Hinweises auf die Patenterteilung: **21.06.2006**
(21) Anmeldenummer: 02737825.6
(22) Anmeldetag: 30.04.2002
(51) Int. Cl.: G01N 27/414, G01N 33/00, G01N 33/497

(54) **VORRICHTUNG UND VERFAHREN ZUR QUANTITATIVEN MESSUNG VON STICKOXIDEN IN DER AUSATEMLUFT UND VERWENDUNG**
DEVICE AND METHOD FOR THE QUANTITATIVE DETERMINATION OF NITROGEN OXIDES IN EXHALED AIR AND APPLICATION THEREOF
DISPOSITIF ET PROCEDE DE MESURE QUANTITATIVE D'OXYDES D'AZOTE CONTENUS DANS DE L'AIR EXPIRE ET LEUR UTILISATION

(30) Priorität: 30.04.2001 DE 10121262
(43) Veröffentlichungstag der Anmeldung: 28.01.2004
(73) Patentinhaber: SIEMENS AKTIENGESELLSCHAFT, 80333 München (DE)
(72) Erfinder: ABRAHAM-FUCHS, Klaus, 91058 Erlangen (DE); FLEISCHER, Maximilian, 85635 Höhenkirchen (DE); MEIXNER, Hans, 85540 Haar (DE); RUMPEL, Eva, 91052 Erlangen (DE); SIMON, Elfriede, 80639 München (DE)
(86) Internationale Anmeldenummer: PCT/DE2002/001576
(87) Internationale Veröffentlichungsnummer: WO 2002/088691

(56) Entgegenhaltungen:
- US-A- 5 431 883
- LAMPE U ET AL: "Nitrogen oxide sensors based on thin films of BaSnO3" SENSORS AND ACTUATORS B, ELSEVIER SEQUOIA S.A., LAUSANNE, CH, Bd. 26, Nr. 1-3, 1995, Seiten 97-98, XP004318432 ISSN: 0925-4005
- ROBINSON J K ET AL: "LUMINOL/H2O2 CHEMILUMINESCENCE DETECTOR FOR THE ANALYSIS OF NITRIC OXIDE IN EXHALED BREATH" ANALYTICAL CHEMISTRY, AMERICAN CHEMICAL SOCIETY. COLUMBUS, US, Bd. 71, Nr. 22, 15. November 1999 (1999-11-15), Seiten 5131-5136, XP000926903 ISSN: 0003-2700
- LEU M ET AL: "EVALUATION OF GAS MIXTURES WITH DIFFERENT SENSITIVE LAYERS INCORPORATED IN HYBRID FET STRUCTURES" SENSORS AND ACTUATORS B, ELSEVIER SEQUOIA S.A., LAUSANNE, CH, Bd. B18, 1994, Seiten 678-681, XP000861740 ISSN: 0925-4005
- CANTALINI C ET AL: "Cross sensitivity and stability of NO2 sensors from WO3 thin film" SENSORS AND ACTUATORS B, ELSEVIER SEQUOIA S.A., LAUSANNE, CH, Bd. 35, Nr. 1, 1. September 1996 (1996-09-01), Seiten 112-118, XP004049740 ISSN: 0925-4005
- BARKER P S ET AL: "A hybrid phthalocyanine/silicon field-effect transistor sensor for NO2" THIN SOLID FILMS, ELSEVIER-SEQUOIA S.A. LAUSANNE, CH, Bd. 284-285, 15. September 1996 (1996-09-15), Seiten 94-97, XP004078110 ISSN: 0040-6090
- SHU JI QIN ET AL: "THE SENSITIVITY TO NO2 OF SANDWICH DEVICES BASED ON LEAD PHTHALOCYANINE AND COPPER PHTHALOCYANINE" SENSORS AND ACTUATORS B, ELSEVIER SEQUOIA S.A., LAUSANNE, CH, Bd. B3, Nr. 4, 1. April 1991 (1991-04-01), Seiten 255-260, XP000243207 ISSN: 0925-4005

## Beschreibung

Die Erfindung betrifft eine Vorrichtung und ein Verfahren zur quantitativen Messung von Stickoxiden, insbesondere Stickstoffdioxid, wobei der Ursprungsgehalt von Stickstoffmonoxid berechenbar ist. Weiterhin wird die Verwendung dieser Vorrichtung in Zusammenhang mit Atemwegserkrankungen wie beispielsweise Asthma beschrieben.

Bei etwa 5 % der Erwachsenen und bei 15 bis 20 % der Kinder in westlichen Industrienationen gehört Asthma zu den am häufigsten auftretenden Krankheiten und dies mit steigender Tendenz.

Entzündungszustände der unteren Atemwege wie zum Beispiel Asthma oder Bronchiektasen sind mit einer erhöhten Stickstoffmonoxid (NO) Konzentration von bis zu 85 ppb in der Ausatemluft verbunden. Bei der Behandlung mit entzündungshemmenden Medikamenten wie bei Kortikosteroide wird ein Rückgang der NO-Konzentration beobachtet. Ein bevorstehender Asthmaanfall zeigt sich im Anstieg des NO-Gehaltes der Ausatemluft deutlich früher als in einem Lungenfunktionstest. Stickstoffmonoxid ist somit ein Vorbote für einen Asthmaanfall. Damit bietet sich diese NO-Messung in der Ausatemluft als idealer Ansatz zur Diagnose und vor allem zur Therapieentscheidung und zur Therapieverlaufskontrolle von Asthma und weiteren mit Entzündungen verbundenen Krankheiten der unteren Atemwege.

Voraussetzung für eine Therapieverlaufskontrolle ist jedoch ein mobiles kostengünstiges Messgerät zur quantitativen und ortsunabhängigen Detektion von Stickoxiden. Weiterhin wäre eine ständige telemedizinische Betreuung von chronisch Kranken interessant.

Nötige Messgenauigkeiten bei der Stickoxiddetektion in der Ausatemluft im Zusammenhang mit den erwähnten Krankheiten liegen im Bereich von wenigen ppb NO. Diese wurden bisher lediglich mit Chemolumineszenzmessungen erreicht. Nachteile bestehen zum einen in der Größe, womit ein Gewicht von mindestens 45 kg verbunden ist und den hohen Kosten einer solchen Chemolumineszenz-Messapparatur. Diese werden nach wie vor für den Einsatz in Kliniken und in Spezialpraxen beschränkt sein.

Zur Verlaufskontrolle von beispielsweise Asthma werden zur Zeit sogenannte Peak-Flow-Meter eingesetzt. Dies sind kleine Geräte, die einen einfachen Lungenfunktionstest erlauben. Im Gegensatz zur vollständigen Lungenfunktionsprüfung wird nur die höchste Atemstromstärke gemessen, der sogenannte Atemstoßwert. Dieser wird bei einer maximalen Ausatmung erzeugt. Die Messung erfolgt mehrmals täglich. Das Gerät ist relativ kostengünstig. Hierbei wird jedoch das Endergebnis eines Asthmaanfalles, nämlich die Verengung der Atemwege, gemessen und nicht ein Vorbote des Anfalles, wie es beispielsweise das Stickstoffmonoxid wäre. Somit verstreicht wertvolle Zeit für eine vorbeugende Behandlung.

Weitere Erkenntnisse über den Entzündungszustand der unteren Atemwege können auch aus dem Bronchialsekret, dem Sputum, gewonnen werden. Die allgemeine Sputumuntersuchung erfolgt makroskopisch, mikroskopisch und bakteriologisch und ist vergleichsweise zeitaufwendig. Die Gewinnung des Sekrets ist für Kinder und Patienten mit starken Atembeschwerden kaum oder gar nicht zu bewerkstelligen.

Aus Robinson, J. K. et al, "Luminol/H₂O₂ Chemiluminescence Detector for the Analysis of Nitric Oxide in Exhaled Breath", Analytical Chemistry, American Chemical Society. Columbus, Us, Bd. 71, Nr. 22, 15. November 1999 (1999-11-15), Seiten 5131 - 5136, ISSN: 0003-2700, ist ein Detektor zur Ermittlung von Stickoxiden bekannt, der zunächst Stickstoffmonoxid zu Stickstoffdioxid aufoxidiert und anschließend durch ein Chemoluminiszenzverfahren Stickstoffdioxid misst. Es werden Varianten angegeben, mit denen die Sensitivität auf Stickstoffdioxid verbessert werden kann.

Aus der Druckschrift Leu et al., "Evaluation of Gas Mixtures with different sensitive Layers incorporated in Hybrid FET Structures", Sensors and Aktuators B, Elsevier Sequoia S.A., Lausanne, CH, Bd.B18, 1994, Seiten 678-681, ISSN: 0925-4005, ist eine hybride Technologie für Gassensoren bekannt, wobei gassensitive Schichten Feldeffekttransistoren beispielsweise aus Metall oder aus Oxiden bestehen. Das Prinzip der Messung der Austrittsarbeitänderung erlaubt eine Detektion von Wasserstoff oder Ammoniak oder Stickstoffdioxid oder Kohlenmonoxid.

Der Erfindung liegt die Aufgabe zugrunde, ein einfaches Messsystem zur quantitativen und möglichst ortsunabhängigen Messung von Stickoxiden und ein Betriebsverfahren bereitzustellen, womit bei der Kontrolle von Atemwegserkrankungen der Stickstoffmonoxidgehalt in der Ausatemluft detektierbar ist.

Die Lösung dieser Aufgabe geschieht durch die jeweilige Merkmalskombination der Ansprüche 1, 19 bzw. 22.

Vorteilhafte Ausgestaltungen können den Unteransprüchen entnommen werden.

Der Erfindung liegt die Erkenntnis zugrunde, dass die mobile kostengünstige quantitative Messung von Stickoxiden in der Ausatemluft mit einer Vorrichtung erzielbar ist, die mit einer Einrichtung zur Führung des Volumenstroms der Ausatemluft der Reihe nach einen Oxidationskatalysator zur Oxidation von Stickstoffmonoxidgehalten zu Stickstoffdioxid aufweist, sowie weiterhin eine Gassensoreinheit zur Detektion von Stickstoffdioxid, Feuchtigkeit und Ethanol und eine Einheit zur Berechnung der Stickstoffmonoxidwerte aus den Stickstoffdioxidwerten, wobei die Feuchtigkeits- und Ethanol-Konzentrationen Querempfindlichkeiten der Stickoxidmessungen eliminieren. Die Kombination aus einem sehr genauen Stickoxidmessverfahren, mit dem auch quantitative Messungen von Stickoxiden in der Ausatemluft möglich sind, mit einem geeigneten Messaufbau, der die Vorverarbeitung des Messgasgemisches gewährleistet zusammen mit der Berechnung des Stickstoffmonoxidanteils in der Ausatemluft ergibt ein kostengünstiges Gassensorsystem zur Feststellung des Stickstoffmonoxidgehaltes und lässt Rückschlüsse auf Atemwegserkrankungen zu.

Zur Stickoxiddetektion einen Gassensor nach dem Prinzip der Austrittsarbeitsmessung einzusetzen ist energetisch vorteilhaft. Dadurch lassen sich Messungen mit relativ geringem Heizenergiebedarf durchführen, was die Entwicklung eines kostengünstigen Sensors erleichtert. Weiterhin können Anwendungen mit Sensoren erschlossen werden, deren elektrische Leistung umgebungsbedingt niedrig sein müssen. Darüber hinaus bietet der Einsatz dieses Messprinzips die Vorteile einer relativ breiten Palette von sensitiven Materialien, die relativ einfach präparierbar sind.

Eine vorteilhafte Ausgestaltung der Erfindung sieht vor, dass der Volumenstrom der Ausatemluft in der Vorrichtung geteilt wird, wobei ein Teilvolumenstrom über den Oxidationskatalysator und danach zur Stickoxidmessung und ein anderer Teilvolumenstrom direkt zur Stickoxidmessung geführt wird. Hierdurch kann insbesondere der Nachweis von Stickstoffmonoxid in der Ausatemluft ohne Störungen durch Stickoxide aus der Umgebungsluft ermittelt werden. Durch die Differenz der Stickstoffdioxidkonzentration kann der eigentliche Stickstoffmonoxidgehalt der Ausatemluft fehlerfrei bestimmt werden. Die Volumenstrommessung geschieht durch übliche Messverfahren.

Bei Einsatz der Stickoxiddetektion nach dem Prinzip der Austrittsarbeitsmessung sind verschiedene Feldeffekttransistoren bekannt, bei denen die gassensitive Schicht als Gate-Elektrode dargestellt ist. Diese Gate-Elektrode kann durch einen Luftspalt abgetrennt sein von dem sogenannten Kanalbereich des Feldeffekttransistors. Grundlage für ein detektierendes Messsignal ist die Änderung des Potentials zwischen Gate und Kanalbereich (ΔV_{G}). In den deutschen Patentanmeldungen Nr. 198 14 857.7 und Nr. 199 56 806.5 werden beispielsweise hybride Flip-Chip-Aufbauten von Gassensoren beschrieben, die als CMOS-Transistoren ausgeführt sind. Ein Gassensor kann darüber hinaus mit zwei Feldeffekttransistoren bestückt sein, deren Regelverhalten durch annähernd gleichgroße Luftspalte zwischen Kanalbereich und Gateelektrode angeglichen ist und deren Sensorschichten separat auslesbar sind. In der deutschen Patentanmeldung Nr. 199 56 744.1 wird beschrieben wie die Beabstandung zwischen Gate-Elektrode und Kanalbereich eines Feldeffekttransistors durch äußerst präzise Abstandshalter reproduzierbar darstellbar ist. Eine andere Ausgestaltung sieht vor, das gassensitive Material in poröser Form auf dem Kanalbereich oder dem Gate aufzubringen.

Durch die Kombination von mehreren Einzelsensoren in einer Gassensoreinheit in Form von verschiedenen gassensitiven Schichten können Querempfindlichkeiten eliminiert werden. Eine Schicht muss selbstverständlich für das Zielgas sensitiv sein. Weitere gassensitive Schichten sind auf die Detektion von Feuchtigkeit bzw. von Alkohol ausgelegt. Eine Referenzschicht ist beispielsweise insensitiv. Eine Integration derart unterschiedlicher gassensitiver Schichten in einer Gassensoreinheit ermöglicht die Eliminierung von Feuchtigkeits- und Alkoholeinflüsse. Zusätzlich muss, um Messstörungen durch den Einfluss der von der Sensortemperatur abweichenden Ausatemtemperatur bzw. Messgastemperatur zu eliminieren, eine Temperaturkompensation mit einem zweiten Transistor durchgeführt. Bei der selektiven Detektion von Ausatemgasen muss besonders der Einfluss der Feuchtigkeit berücksichtigt werden, da die Konzentration der Feuchte bis zu 100 % relativer Luftfeuchtigkeit ansteigen kann. Außerdem ist es sinnvoll, die Konzentration von Alkohol zu berücksichtigen, da diese Komponente im Ausatemgas im Vergleich zu Stickoxiden in sehr hohen Konzentrationen auftreten kann.

Eine Schichtkombination sieht beispielsweise wie folgt aus: Zielgas-sensitive Schicht , Gas-insensitive Referenzschicht, Feuchte-sensitive Schicht, Feuchte-insensitive Referenzschicht, Alkohol-sensitive Schicht, Alkohol-insensitive Schicht und Temperatursensor in einer Einheit. Somit können Messstörungen durch Einfluss der von der Sensorbetriebstemperatur abweichenden Temperatur der Ausatemluft, Feuchteeinflüsse sowie Einflüsse durch im Atem enthaltenen Alkohol eliminiert werden.

Gassensitive Schichten zum Einsatz in einem sogenannten SG-FET (Suspended Gate-Feldeffekttransistor) sind Porphinfarbstoffe, wie z.B. Phthalocyanine mit dem Zentralatom Kupfer oder Blei. Bei Sensortemperaturen zwischen 50° und 120° C können Stickoxidsensitivitäten bis in den unteren ppb-Bereich nachgewiesen werden. Die Detektion zielt wie üblich auf Stickstoffdioxid ab, wobei Stickstoffmonoxid nach der bisher beschriebenen Methode errechenbar ist.

Zur Detektion von Ethanol sind gassensitive Schichten aus Polysiloxanen ebenfalls denkbar. Feuchtigkeit wird vorzugsweise mit gassensitiven Schichten aus Polyamid oder Polypyrrolidon detektiert.

Phthalocyanine eignen sich besonders gut für ΔΦ-Messungen zur Detektion von NO₂-Gasen mit den Zentralatomen wie Kupfer oder Blei aber auch die Verbindungen die Zinn, Nickel, Cobalt oder Zink als Zentralatom aufweisen. Ganz besonders NO2-empfindlich sind Phthalocyaninverbindungen und deren Derivate die kein Zentralatom besitzen; stattdessen sind die freien Bindungsstellen im Porphinring mit Wasserstoffatomen abgesättigt wie z.B. bei Heliogen Blau G und ein Phthalocyanin mit Phenyletherseitenketten. Bei Sensortemperaturen zwischen Raumtemperatur und 120°C können NO₂-Sensitivitäten bis in den unteren ppb-Bereich nachgewiesen werden.

Zum Nachweis von NO₂ im unteren ppb-Bereich eignen sich, neben den Phthalocyaninfarbstoffen auch Porphyrine und Metalloporphyrine. Hierzu zählen die metallfreien Porphyrine wie z.B. das Protoporphyrin IX Natriumsalz oder metallhaltige Porphyrine wie das Cobalt-Protoporphyrine IX. Um die Ansprechzeiten der Sensoren zu verbessern werden die Sensorschichten bei Temperaturen von Raumtemperatur bis zu 75°C verwendet.

Vorteil dieser untersuchten Materialien ist, im Vergleich zu den Phthalocyaninen, dass sich bereits bei relativ niedrigeren Temperaturen sehr kleine Ansprechzeiten ergeben und eine hohe NO₂-Ernfindlichkeit vorliegt. Deshalb können auch die Sensoren bei niedrigerer Heizspannung betrieben werden, was den Energieverbrauch des Sensors deutlich verringert.

Im Folgenden werden anhand von schematischen Figuren Ausführungsbeispiele beschrieben:
- Figur 1: zeigt eine NO₂-Kennlinie eines Sensors mit einer gas-sensitiven Schicht aus Kupfer-Phthalocyanin,
- Figur 2: zeigt einen Gassensor nach dem Prinzip der Austrittsarbeitsmessung als Suspended-Gate-FET,
- Figur 3: zeigt den Aufbau eines Sensorsystems zur Bestimmung der Stickstoffmonoxidkonzentration in der Ausatemluft mit der Korrektur von Querempfindlichkeiten, beispielsweise aus der Umgebungsluft,
- Figur 4: zeigt das Prinzip der Konvertierung von Stickstoffmonoxid zu Stickstoffdioxid,
- Figur 5: zeigt ein gesamtes Sensorsystem zur Bestimmung der Stickstoffmonoxidkonzentration in der Ausatemluft,
- Figur 6: zeigt die Möglichkeiten der Umsetzung einer Stickstoffmonoxidmessung für die Diagnose, Therapieplanung und Verlaufskontrolle bei Asthmaerkrankungen,
- Figur 7: zeigt ein Diagramm mit einer NO₂-Kennlinie von Heliogen Blau G und einer von einem Phenyletherderivat eines H2-Phthalocyanins,
- Figur 8: zeigt ein Diagramm mit einer NO₂-Kennlinie von Co-Protoporphyrin und einer von metallfreiem Protoporphyrin Na-Salz,
- Figur 9: zeigt den Aufbau eines Probenahmesystems zur Verwendung in einem Asthmasensor,
- Figur 10: zeigt ein weiteres Beispiel eines Sensorsystems zur Bestimmung der NO-Konzentration in der Ausatemluft.

Zur Bestimmung der Stickoxide in der Ausatemluft zur Detektion von Asthma muss gewährleistet sein, dass nur die bronchial ausgeatmete Luft detektiert wird. Da Luft, die über die Nase ausgeatmet wird, um den Faktor 1000 höhere Stickoxidkonzentrationen aufweist, muss das zu messende Atemluftvolumen genau definiert werden. Dies kann dadurch erreicht werden, dass gegen einen Widerstand ausgeblasen wird, beispielsweise über ein nur bei erhöhtem Luftdruck öffnendes Ventil, so dass sich automatisch das Gaumensegel schließt und der Atem ausschließlich durch den Mund ausgeblasen wird und nicht über die Nase.

Zum Nachweis von Stickstoffmonoxid in der Ausatemluft muss eine Störung durch Stickoxide aus der Umgebungsluft ausgeschlossen werden. Dazu wird der Volumenstrom der Ausatemluft geteilt. Ein Teil der NO-haltigen Ausatemluft wird direkt zur Bestimmung der NO₂-Rest-Konzentration verwendet. Der zweite Teil wird mittels Konverter (Oxidationskatalysator) zu Stickstoffdioxid oxidiert und anschließend wird die Stickstoffdioxidkonzentration gemessen. Durch die Differenz der Stickstoffdioxidkonzentrationen kann der eigentliche Stickstoffmonoxidgehalt der Ausatemluft genauer bestimmt werden.

Wesentliche Vorteile des Gesamtsystems liegen darin, dass eine nicht invasive Messmethode verwendet wird. Die Messungen sind in großer Anzahl wiederholbar und können somit auch zur Verlaufskontrolle bei Therapien, bei der Diagnose von Asthma bei Kindern, bei der Früherkennung von Asthma oder für vorbeugende medizinische Maßnahmen besonders eingesetzt werden. Das hier vorgestellte System mit einer Gassensoreinheit ist für die Herstellung kleiner und kostengünstiger Atemgassensoren verwendbar und ist deshalb auch für den Einsatz außerhalb von Kliniken und Arztpraxen geeignet.

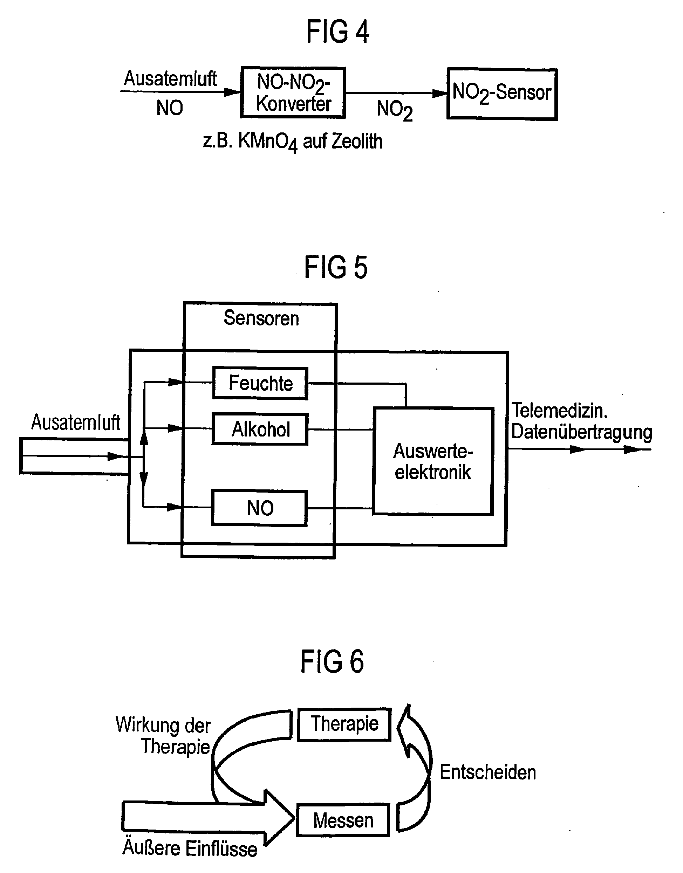

Die Kette der Aktionen zur Veranschaulichung des Anwendungsszenarios beschreibt **Figur 6.** Am Beispiel von Asthma kann ausgeführt werden, dass Messungen der Stickstoffmonoxidkonzentration als Schlüssel zur Diagnose Therapieplanung und Verlaufskontrolle einen wesentlichen Beitrag für die Entscheidungsfindung liefern. Weitere Vorteile des erfindungsgemäßen Aufbaus liegen in der Eliminierung von Querempfindlichkeiten bzw. Messstörungen durch Stickoxidgase aus der Umgebungsluft während der Bestimmung der Stickstoffmonoxidkonzentration.

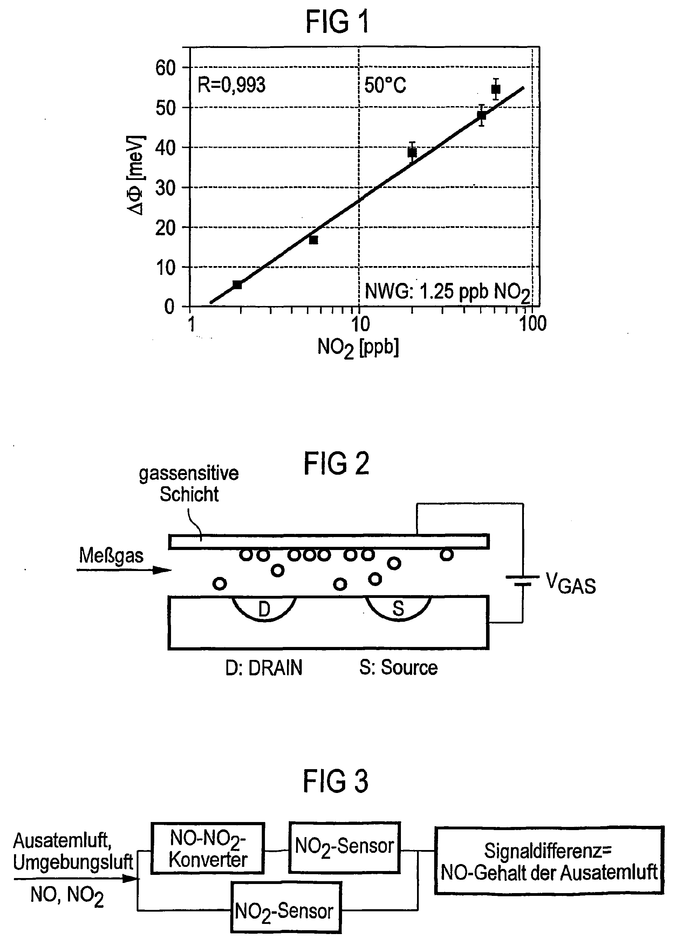

Durch das Auftreten von Stickoxiden in der Umgebungsluft in Konzentrationsbereichen, die für den Nachweis einer Asthmaerkrankung relevant sind, müssen Störeffekte aus der Umgebungsluft eliminiert werden. Dazu wird die Stickstoffdioxidkonzentration, die sich bereits in der Ausatemluft befindet oder eventuell durch Probenahmefehler in die Ausatemluft gelangt, mittels Stickstoffdioxidgassensoren direkt bestimmt. Parallel dazu wird der Stickstoffmonoxidgehalt der Ausatemluft mittels eines Konverters quantitativ in Stickstoffdioxid umgewandelt und mit einem zweiten Stickstoffdioxidsensor quantifiziert. Das Differenzsignal dieser beiden Messungen ergibt schließlich den Gehalt an Stickstoffmonoxid in der Ausatemluft und einen Nachweis zur Beurteilung der Asthmaerkrankung. In Figur 3 wird schematisch der beschriebene Verfahrensablauf dargestellt.

**Figur 4** zeigt ein Schema für die Konvertierung von Stickstoffmonoxid zu Stickstoffdioxid. Um die Stickstoffmonoxidkonzentration der Ausatemluft bestimmen zu können, wird der Gehalt an Stickstoffmonoxid quantitativ zu Stickstoffdioxid umgewandelt und mittels eines Stickstoffdioxidsensors wird die Konzentration bestimmt. Zur Konvertierung des Messgases wird ein Oxidationsmittel wie beispielsweise Permanganatsalze oder Perchloratsalze verwendet, das in der Regel auf einem Katalysatorträger wie beispielsweise Zeolith, Tonerde oder Kieselgel aufgebracht ist. Dieser Katalysator wird in den Gasstrom des Ausatemgases gebracht, um so das in der Ausatemluft enthaltene Stickstoffmonoxid quantitativ zu Stickstoffdioxid umzuwandeln. Dieses NO₂-Gas wird mittels hochsensitiver NO₂-Gassensoren detektiert. Der NO₂-Gehalt des Gases entspricht dem NO-Gehalt der Ausatemluft.

Durch die Eliminierung von Querempfindlichkeiten geht eine Verbesserung der Messgenauigkeit einher. Das Sensorsystem zum Nachweis einer Asthmaerkrankung ist mit mindestens drei Sensoren ausgestattet. Detektiert werden das Zielgas Stickoxid, die Feuchtigkeit und Ethanol. Mit einer Auswerteelektronik und Übertragung der Daten in ein telemedizinisches Netz ergeben sich besondere Vorteile hinsichtlich der Asthmaerkennung und Behandlung.

Die Bestimmung der Feuchtekonzentration und die direkte Signalkorrektur mit dem NO-Sensorsignal ist notwendig, um die Querempfindlichkeit der NO-Sensorschicht auf Feuchte zu eliminieren. Die Bestimmung der Alkoholkonzentration in der Ausatemluft ist zur Qualitätsbeurteilung der NO-Messung erforderlich, da Alkohol in der Ausatemluft bis zu einer Höhe von 1500 ppm auftreten kann und zu einer Verfälschung der NO-Detektion im unteren ppb-Bereich führen kann. In **Figur 5** ist ein Beispiel eines Sensorsystems zur Bestimmung der NO-Konzentration in der Ausatemluft dargestellt. Die Ausatemluft wird in diesem Fall nicht über eine Serie von verschiedenen Sensoren geleitet, sondern in verschiedene Volumenströme geteilt und entsprechenden unterschiedlichen Sensoren zugeführt. Hierzu sind verschiedene Volumenstrommessungen notwendig, um auf den Teilgasgehalt in der Ausatemluft rückrechnen zu können. Jede Stufe zur NO-Detektion in **Figur 5** beinhaltet entsprechende Funktionseinheiten zur Konvertierung in NO₂ und dessen Detektion. Weiterhin kann über einen weiteren in dieser NO-Stufe enthaltenen Bypass die wie in **Figur 3** dargestellte NO₂-Grundkonzentration in der Atemluft festgestellt werden.

Eine Vernetzung der Ausgangssignale des durch diese Erfindung beschriebenen Systems ermöglicht ein sogenanntes Asthma-Monitoring, wobei die Stickstoffmonoxiddetektion in der Ausatemluft als wesentlicher Teil eingeht. Damit verbundene Vorteile sind einfache ortsunabhängige Handhabung, sowie die Durchführbarkeit regelmäßiger reproduzierbarer Messungen. Die dadurch gewonnenen lückenlosen Verlaufsdaten liefern Hinweise auf Anfall auslösende Faktoren oder systematisches Patientenfehlverhalten. Da sich in den NO-Werten der Ausatemluft eine Veränderung des Krankheitszustandes frühzeitig ankündigt, ermöglicht die zeitnahe Übermittlung der Daten an den behandelnden Arzt eine rechtzeitige medizinische Anpassung was mittelfristig eine Minimierung der Medikamenteneingabe zur Folge hat.

**Figur 1** zeigt die Austrittsarbeitsänderung in Abhängigkeit von dem Stickstoffdioxidgehalt beim Einsatz einer gassensitiven Schicht aus Kupfer-Phthalocyanin.

**Figur 2** zeigt das Konzept eines gassensitiven Feldeffekttransistors, der mit einer auf der zum Kanalbereich beabstandeten Gate-Elektrode aufgebrachten gassensitiven Schicht ausgestattet ist. Die Auslesung eines Signals an der gassensitiven Schicht geschieht nach dem Prinzip der Austrittsarbeitsmessung bzw. nach der Austrittsarbeitsänderung.

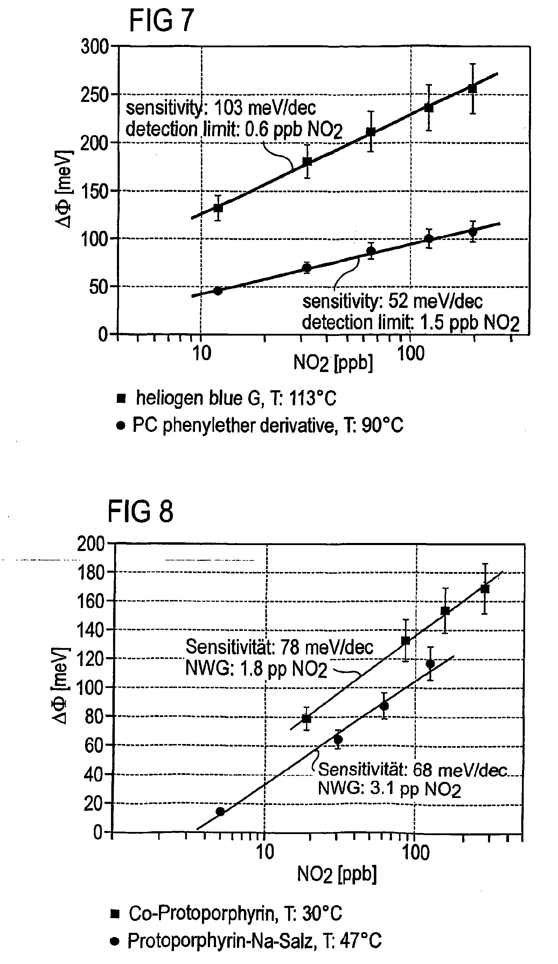

Die **Figuren 7 und 8** zeigen Beispiele bestimmter NO-Sensoren mit gezielt ausgewählten NO-sensitiven Stoffen als gassensitive Schicht im Sensor.

Vorteile der Erfindung ergeben sich besonders in der Anwendung:
Die NO-Bestimmung im Ausatemgases ist eine nicht invasive Messmethode, die für wiederholte Anwendungen wie z. B. die Verlaufkontrolle bei Therapien, bei der Diagnose von Asthma bei Kindern, bei der Früherkennung von Asthma oder für vorbeugende medizinische Maßnahmen besonders geeignet ist. Verfälschungen der Messung durch Veränderungen der Temperatur an Sensoren oder durch vorhandene Feuchte können korrigiert werden.
Störungen durch hohe Ethanolkonzentrationen können erkannt und korrigiert werden.
Das Gassensorsystem kann für beliebig viele, wiederholbare Messungen in der Ausatemluft verwendet werden.
Der vorgestellte Gassensor ist für die Herstellung kleiner und kostengünstiger Atemgassensoren verwendbar und ist deshalb auch für den Einsatz außerhalb von Kliniken und Arztpraxen geeignet.

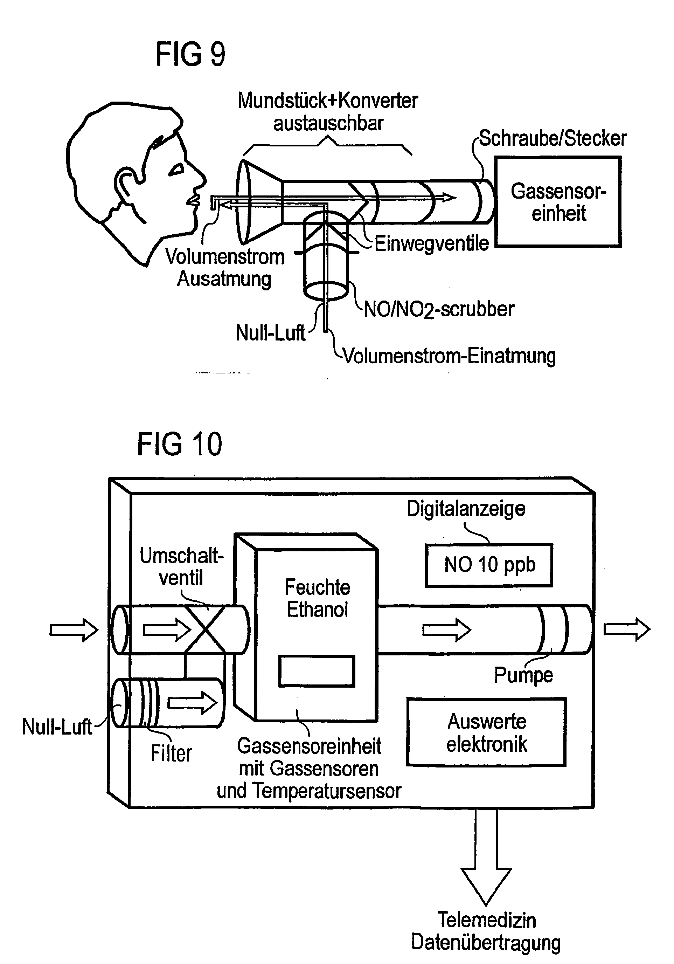

Aufbau eines Meßsystems zur Bestimmung von NO in der Ausatemluft:
Das Meßsystem besteht aus einem Probenahmesystem und aus einem Gassensorsystem.
Das Probenahmesystem, wie es in **Figur 9** erkennbar ist, besteht aus einem Mundstück aus biokompatiblem Material, dem NO/NO₂-Konverter zur Aufoxidation und einem NO/NO₂-Scrubber zur NO/NO₂-Eliminierung aus dem Messgas. Dieses Probenahmesystem ist flexibel an das Gassensorsystem ankoppelbar, beispielsweise mit Schraubverschluss oder Stecker und kann nach ein- oder mehrmaligem Benutzen erneuert werden.
Mit Hilfe des Probenahmesystems wird vor der Messung mittels eines NO/NO₂-Scrubbers Null-Luft, also NO- und NO₂-freie Luft, eingeatmet. Der NO/NO₂-Scrubber besteht aus einer Filterkombination von Aktivkohle und Aluminiumoxid bzw. Zeolith oder Kieselgel bzw. der Kombination dieser Materialien. Dieser NO/NO₂-Scrubber ist mit einem Einwegeventil ausgestattet, so dass nur durch den NO/NO₂-Scrubber eingeatmet werden kann und nicht über die Konvertersäule. Mit dieser Anordnung wird verhindert, dass NO- oder NO₂ -Gas, welches in der Umgebungsluft in wesentlich höheren Konzentrationen vorliegt als in der Lunge, durch das Einatmen in die Lunge gelangt und so in der Ausatemluft erhöhte Werte vortäuschen könnte.
Die ausgeatmete Luft wird über ein zweites Einwegeventil zu dem NO/NO₂-Konverter geblasen und nicht durch den NO/NO2-Scrubber.Der Konverter besteht aus Kaliumpermanganat welches auf Kieselgel immobilisiert ist.

Der Konverter dient zur:
- Konvertierung des NO-Gases zu NO₂-Gas bei Raumtemperatur,
- Reduzierung der Feuchte des Ausatemgases.

Vorteile der Erfindung liegen besonders in der Anwendung:
- Es wird NO- und NO₂- freie Luft eingeatmet wodurch die Messung störungsfrei von Einflüssen der Umgebungsluft ist,
- Betauung des Sensors wird vermieden, da Kieselgel einen Teil der Atemfeuchte absorbiert,
- Eine Heizung des Konverters entfällt, da die Oxidation von NO-Gas zu NO₂-Gas bei Raumtemperatur erfolgt,
- Flexibler und schneller Austausch der zu erneuernden Systemteile, wie Mundstück, da es aus hygienischen Gründen erneuert werden muss oder Konverter, der sich verbraucht und ausgetauscht werden muss.

Das Gassensormesssystem entsprechend Figur 10 besteht aus einer Zuführung für das NO₂-haltige Atemgas, welches über ein Umschaltventil in die Gassensoreinheit geblasen wird. In der Gassensoreinheit befinden sich mindestens drei Gassensoren und drei Referenzsensoren zur Bestimmung des NO₂-Gehaltes, der Feuchte und des Ethanolgehaltes in der Ausatemluft. Desweiteren befindet sich dort ein Temperatursensor zur Bestimmung der Gastemperatur. Jeder Sensor besitzt eine Temperaturregelung um die Temperatur der Gassensoren einzustellen. Nach der Gassensoreinheit folgt der Gasauslass mit einer Mikropumpe. Neben dem Gaseinlass für das NO₂-haltige Gas der Atemluft befindet sich ein zweiter Gasseinlass, der mit einem Filtersystem (Aktivkohle + Aluminiumoxid/Zeolith/Kieselgel-Kombination) ausgestattet ist. Dieses Filtersystem dient dazu Null-Luft herzustellen und nach der Gasmessung die Messkammmer/Gassensoreinheit und die Zuführungskanäle von NO₂-Gas und auch anderen Störgasen zu befreien und die Gassensoren zu regenerieren. Mit Hilfe der Micropumpe am Gasauslass und mittels Umschaltventil vor der Messkammer wird Null-Luft, NO/NO₂-freie Luft, angesaugt und das gesamte Sensorsystem regeneriert und wieder in den Messmodus gebracht. Das Gassensorsystem ist weiterhin mit einer Auswerteelektronik bestückt um die Signale der Gassensoren und Referenzsensoren auszulesen, die Feuchtekorrektur durchzuführen, die Gaskonzentration zu berechnen und die NO-Konzentration zu bestimmen. Der NO-Gehalt wird auf einer Digitalanzeige am Messsystem angezeigt und steht auch für die telemedizinische Datenübertragung zur Verfügung.

**Figur 9** zeigt insbesondere die Verbesserung der Messanordnung durch Einbau der Konvertierungssäule und Anordnung einer Null-Luft-Zuführung am Mundstück.

## Patentansprüche

1. Vorrichtung zur quantitativen Ermittlung von Stickstoffmonoxid in einer Ausatemluft durch eine Gassensoreinheit mit mindestens einem Gassensor, wobei ein Oxidationskatalysator zur Oxidation von Stickstoffmonoxid zu Stickstoffdioxid vorhanden ist, **dadurch gekennzeichnet dass** der mindestens eine Gassensor nach dem Prinzip der Austrittsarbeitsmessung betrieben wird und eine gassensitive Schicht aufweist mit einem gassensitiven Material, welches einen Porphinfarbstoff enthält oder durch diesen dargestellt ist, wobei die Gassensoreinheit weitere Sensoren zur Detektion von Feuchtigkeit oder Ethanol zur Eliminierung von Querempfindlichkeiten enthalten kann.

2. Vorrichtung nach Anspruch 1,
bei der zur Auslesung der Austrittsarbeit ein hybrider Feldeffekttransistor eingesetzt ist, in dem die gassensitive Schicht als vom Kanalbereich beabstandete Gate-Elektrode dargestellt ist.

3. Vorrichtung nach einem der Ansprüche 1 oder 2,
bei der zur Auslesung der Austrittsarbeit ein Feldeffekttransistor vorhanden ist, bei dem die gassensitive Schicht in Form von porösem auf dem Kanalbereich abgeschiedenem Material dargestellt ist.

4. Vorrichtung nach einem der Ansprüche 2 - 3,
bei dem Gassensoren Feldeffekttransistoren mit hybridem Flip-Chip-Aufbau sind.

5. Vorrichtung nach einem der vorhergehenden Ansprüche,
bei der in einer Gassensoreinheit zusätzlich eine gasinsensitive Referenzschicht vorhanden ist.

6. Vorrichtung nach Anspruch einem der vorhergehenden Ansprüche,
bei der ein Porphinfarbstoff ein Phthalocyanin oder ein Deri-vat davon ist mit dem Zentralatom Kupfer, Blei, Zinn, Nickel, Kobalt oder Zink, oder ein Phthalocyanin ohne Zentralatom, dessen freie Bindungsstellen im Porphinring durch Wasserstoffatome gesättigt sind.

7. Vorrichtung nach Anspruch 6,
bei der das Phthalocyanin ohne Zentralatom Heliogen Blau G oder ein Phthalocyanin mit Phenyletherseitenketten ist.

8. Vorrichtung nach Anspruch 6,
bei der ein Porphinfarbstoff ein Porphyrin bzw. Metalloporphyrin ist mit dem Zentralatom Kobalt oder ein Porphyrin ohne Zentralatom.

9. Vorrichtung nach Anspruch 8,
bei der das Metalloporphyrin durch Cobalt-Protoporphyrin IX Chlorid oder das Porphyrin ohne Zentralatom durch Protoporphyrin IX Natriumsalz dargestellt ist.

10. Vorrichtung nach einem der Ansprüche 1 - 5,
bei der Materialien für gassensitive Schichten zur StickoxidDetektion feinkristalline Metalloxide wie SnO₂, WO₃, In₂O₃, Salze aus dem Karbonatsystem Bariumkarbonat oder Polysiloxane sind.

11. Vorrichtung nach einem der vorhergehenden Ansprüche,
bei der Materialien für gassensitive Schichten zur Detektion von Feuchtigkeit Polyamide oder Polypyrrolidone und zur Detektion von Ethanol Polysiloxane sind.

12. Vorrichtung nach einem der vorhergehenden Ansprüche,
wobei die Messvorrichtung zur Stickstoffmonoxidbestimmung in der Ausatemluft mobil ist.

13. Vorrichtung nach einem der vorhergehenden Ansprüche,
mit einer Gasführungseinrichtung zur Zuführung des Volumenstromes der Ausatemluft zu der Gassensoreinheit zur Ermittlung von Stickstoffmonoxid oder Stickstoffdioxid und einer oder mehreren Komponenten wie Feuchtigkeit, Ethanol oder Temperatur.

14. Vorrichtung nach Anspruch 13,
bei der in der Gasführungseinrichtung der Volumenstrom der Ausatemluft aufgeteilt ist in einen Teilvolumenstrom über einen Oxidationskatalysator zur Oxidation von Stickstoffmonoxid zu Stickstoffdioxid und anschließend zu der Gassensoreinheit zur Messung der Stickstoffdioxidkonzentration und einen weiteren Teilvolumenstrom der direkt zur Bestimmung der Stickstoffdioxidkonzentration zu einem weiteren Stickstoffdioxid Gassensor geführt ist, wobei mittels der einzelnen Stickstoffdioxidkonzentrationen die Stickstoffmonoxidkonzentration der Ausatemluft berechenbar ist.

15. Vorrichtung nach Anspruch 13,
bei der in der Gasführungseinrichtung der Volumenstrom der Ausatemluft insgesamt über einen Oxidationskatalysator zur Oxidation von Stickstoffmonoxid zu Stickstoffdioxid und anschließend zu einer Gassensoreinheit zur Messung der Stickstoffdioxidkonzentration geführt ist, wobei mittels der Stickstoffdioxidkonzentration die Stickstoffmonoxidkonzentration der Ausatemluft berechenbar ist.

16. Vorrichtung nach einem der Ansprüche 14 oder 15,
bei der als Material für einen Oxidationskatalysator ein oder mehrere Stoffe aus der Gruppe der Permanganatsalze oder Perchloratsalze auf einem Träger wie Zeolith, Aluminiumoxid oder Kieselgel aufgebracht sind.

17. Vorrichtung nach einem der vorhergehenden Ansprüche,
bei der über eine Auswerteelektronik aufbereitete Sensorsignale einem Datennetz zuführbar sind.

18. Vorrichtung nach einem der Ansprüche 13 bis 17,
bei der die Gasführungseinrichtung mit einer festen oder variablen Drosseleinrichtung zur Erzeugung eines Strömungswiderstandes versehen ist.

19. Verfahren zum Betrieb einer Vorrichtung entsprechend einem der Ansprüche 1 - 18, bestehend aus folgenden Schritten:
eine Ausatemluft wird als Messgas über einen Oxidationskatalysator geleitet um NO zu NO₂ zu oxidieren,
das Messgas wird anschließend der Gassensoreinheit zugeführt, worin der NO₂-Gehalt gemessen wird,
aus dem NO₂-Gehalt wird der NO-Gehalt berechnet,
zur Regeneration des Gassensormesssystems wird dieses mit NOₓ-freier Luft gespült.

20. Verfahren nach Anspruch 19,
bei dem NOₓ-freie Luft zum Einatmen bereitgestellt wird um Umwelteinflüsse zu eliminieren.

21. Verfahren nach Anspruch 19 oder 20,
bei dem NOₓ-freie Luft in einem Scrubber bzw. einer Abscheidevorrichtung für NOₓ erzeugt wird.

22. Verwendung einer Vorrichtung zur quantitativen Ermittlung von Stickoxiden in der Ausatemluft entsprechend einem der Ansprüche 1 bis 18 für die Konzentrationsbestimmung von Stickstoffmonoxid in der Ausatemluft.

23. Verwendung nach Anspruch 22,
bei dem Messsignale aus einer Auswerteelektronik der Vorrichtung an ein und innerhalb eines elektronischen Netzwerk übertragbar sind.

24. Verwendung nach einem der Ansprüche 22 oder 23,
wobei die Messvorrichtung zur Detektion von Asthmaerkrankungen oder Entzündungen der Lunge verwendet wird.

## Claims

1. Device for the quantitative measurement of nitrogen monoxide in exhaled air by means of a gas sensor unit with at least one gas sensor, with an oxidization catalyzer for oxidation of nitrogen monoxide to nitrogen dioxide being present, **characterized in that** the at least one gas sensor is operated in accordance with the principle of exit work measurement and features a gas-sensitive layer which contains a porphine pigment or is represented by same, with the gas sensor unit being able to include further sensors for the detection of moisture or ethanol for elimination of cross-sensitivities.

2. Device according to Claim 1,
with a hybrid field effect transistor in which the gas-sensitive layer is represented by a gate electrode spaced apart from the channel area being used to read the exit work.

3. Device according to one of Claims 1 or 2,
with a field effect transistor being present to read the exit work, with a gas-sensitive layer being in the form of porous material deposited on the channel area.

4. Device according to one of Claims 2 - 3,
with the gas sensors being field effect transistors of a hybrid flip-chip construction.

5. Device according to one of the preceding claims,
with a gas-insensitive reference layer additionally present in a gas sensor unit.

6. Device according to one of the preceding claims,
in which a porphine pigment is a phthalocyanine or a derivate of same, with the central atom being copper, lead, tin, nickel, cobalt or zinc, or a phthalocyanine without a central atom whose free binding sites in the porphine ring are saturated by hydrogen atoms.

7. Device according to Claim 6,
with the phthalocyanine without a central atom being heliogen blue G or a phthalocyanine with phenylether side chains.

8. Device according to Claim 7,
with one of the porphine pigments being a porphyrine or metalloporphyrine with cobalt as a central atom or a porphyrine without a central atom.

9. Device according to Claim 8,
with the metaloporphyrine being represented by cobalt-protoporphyrine IX chloride or the porphyrine without a central atom by protoporphyrine IX sodium salt.

10. Device according to one of Claims 1 - 5,
with the materials for gas-sensitive layers for nitrogen oxide detection being fine crystalline metal oxides such as SnO₂, WO₃, In₂O₃ salts from the carbonate system barium carbonate or polysiloxanes.

11. Device according to one of the preceding claims,
with the material for gas-sensitive layers for detection of moisture being polyamides or polypyrrolidones and being polysiloxanes for the detection of ethanol.

12. Device according to one of the preceding claims,
with the measuring device for determining the nitrogen monoxide in exhaled air being mobile.

13. Device according to one of the preceding claims,
with a gas flow device for the supply of the volumetric flow of nitrogen dioxide or nitrogen dioxide and one or more components such as moisture, ethanol or temperature.

14. Device according to Claim 13,
with the volumetric flow of the exhaled air being divided in the gas flow device into a part volumetric flow via an oxidation catalyst for oxidation of nitrogen monoxide to nitrogen dioxide and then to the gas sensor unit for measurement of the nitrogen dioxide concentration, and into a further part volumetric flow that is supplied directly to a further nitrogen dioxide gas sensor for determining the nitrogen dioxide concentration, with it being possible to calculate the nitrogen monoxide concentration of the exhaled air by means of the individual nitrogen dioxide concentrations.

15. Device according to Claim 13,
with the complete volumetric flow of the exhaled air in the gas flow device being supplied through an oxidation catalyst for oxidation of nitrogen monoxide to nitrogen dioxide and then to a gas sensor unit for measurement of the nitrogen dioxide concentration, with it being possible to calculate the nitrogen monoxide concentration of the exhaled air by means of the nitrogen dioxide concentration.

16. Device according to one of Claims 14 or 15,
with one or more substances from the group of permanganate salts or perchlorate salts being applied to a support such as zeolite, aluminium oxide or silica gel as the material for an oxidation catalyst.

17. Device according to one of the preceding claims,
with it being possible to supply sensor signals processed in an analytical circuit to a data network.

18. Device according to one of Claims 13 to 17,
with the gas flow device being provided with a fixed or variable restrictor device for generation of a flow resistance.

19. Method for operation of a device corresponding to one of Claims 1 - 18, consisting of the following steps:
Exhaled air is supplied as a measured gas through an oxidation catalyst to oxidize NO to NO₂.
The measured gas is then applied to the gas sensor unit where the NO₂ content is measured.
The NO content is calculated from the NO₂ content.
This is purged with nitrogen dioxide-free air for regeneration of the gas sensor measuring system.

20. Method according to Claim 19,
with nitrogen-dioxide-free air being processed for inhalation to elimination environmental influences.

21. Method according to Claim 19 or 20,
with nitrogen-dioxide-free air being generated in a scrubber or separator device for NOₓ.

22. Use of a device for quantitative measurement of nitrogen oxides in exhaled air corresponding to one of Claims 1 to 19 for determining the concentration of nitrogen monoxide in the exhaled air.

23. Use according to Claim 22,
with the measured signals from an analytical circuit of the device being transmittable to and within an electronic network.

24. Use according to one of Claims 22 or 23,
with the measuring device being used to detect asthma illnesses or inflammation of the lungs.

## Revendications

1. Dispositif destiné à la détermination quantitative de monoxyde d'azote dans de l'air expiré, à l'aide d'une unité de détection de gaz comprenant au moins un détecteur de gaz, en présence d'un catalyseur d'oxydation destiné à l'oxydation du monoxyde d'azote en dioxyde d'azote, **caractérisé en ce que** le au moins un détecteur de gaz fonctionne selon le principe de la mesure du travail d'extraction et présente une couche sensible aux gaz comprenant une substance sensible aux gaz qui contient un colorant à base de porphine ou qui est constituée par celui-ci, l'unité de détection de gaz pouvant comprendre d'autres détecteurs destinés à la détection d'humidité ou d'éthanol afin d'éliminer les sensibilités transversales.

2. Dispositif selon la revendication 1,
dans lequel un transistor à effet de champ hybride sert à l'évaluation du travail d'extraction, la couche sensible aux gaz étant constituée par une grille éloignée de la zone de canal.

3. Dispositif selon la revendication 1 ou 2,
dans lequel un transistor à effet de champ sert à l'évaluation du travail d'extraction, la couche sensible aux gaz étant constituée par un matériau poreux précipité sur la zone de canal.

4. Dispositif selon l'une des revendications 2 à 3,
dans lequel les détecteurs de gaz sont des transistors à effet de champ munis de structures Flip-Chip hybrides.

5. Dispositif selon l'une quelconque des revendications précédentes,
dans lequel une unité de détection de gaz comprend en outre une couche de référence insensible aux gaz.

6. Dispositif selon l'une quelconque des revendications précédentes,
dans lequel un colorant à base de porphine est une phtalocyanine ou un de ses dérivés, dont l'atome central est constitué par un atome de cuivre, de plomb, d'étain, de nickel, de cobalt ou de zinc, ou une phtalocyanine dépourvue d'atome central, les sites de liaison libres du cycle porphinique étant saturés avec des atomes d'hydrogène.

7. Dispositif selon la revendication 6,
dans lequel la phtalocyanine dépourvue d'atome central est l'Heliogen Blau G ou une phtalocyanine comportant des chaînes latérales phényléther.

8. Dispositif selon la revendication 6,
dans lequel un colorant à base de porphine est une porphyrine ou une métalloporphyrine ayant un atome central de cobalt ou une porphyrine sans atome central.

9. Dispositif selon la revendication 8,
dans lequel la métalloporphyrine est constituée par le chlorure d'une cobaltoprotoporphyrine IX, ou la porphyrine sans atome central est constituée par le sel de sodium de la protoporphyrine IX.

10. Dispositif selon l'une quelconque des revendications 1 à 5,
dans lequel les substances comprises dans les couches sensibles aux gaz destinées à la détection d'oxydes d'azote comprennent des oxydes métalliques finement cristallisés tels que SnO₂, WO₃, In₂O₃, des sels appartenant au groupe de carbonates comprenant le carbonate de baryum, ou des polysiloxanes.

11. Dispositif selon l'une quelconque des revendications précédentes,
dans lequel les substances pour les couches sensibles aux gaz destinées à la détection de l'humidité sont constituées par un polyamide ou une polypyrrolidone et celles destinées à la détection d'éthanol sont constituées par des polysiloxanes.

12. Dispositif selon l'une quelconque des revendications précédentes,
dans lequel le dispositif de mesure destiné à la détermination du monoxyde d'azote dans l'air expiré est mobile.

13. Dispositif selon l'une quelconque des revendications précédentes,
comprenant un dispositif de guidage de gaz destiné à amener le flux d'air expiré à l'unité de détection de gaz pour déterminer le monoxyde d'azote ou le dioxyde d'azote et un ou plusieurs composants tels que l'humidité, l'éthanol ou la température.

14. Dispositif selon la revendication 13,
dans lequel, à l'intérieur du dispositif de guidage de gaz, le flux d'air expiré est subdivisé en un flux partiel passant sur un catalyseur d'oxydation qui oxyde le monoxyde d'azote en dioxyde d'azote et arrivant dans l'unité de détection de gaz qui mesure la concentration de dioxyde d'azote, et en un autre flux partiel qui est amené directement à un autre détecteur de gaz mesurant la concentration de dioxyde d'azote, la concentration de monoxyde d'azote de l'air expiré pouvant être calculée à partir des concentrations individuelles de dioxyde d'azote.

15. Dispositif selon la revendication 13,
dans lequel, à l'intérieur du dispositif de guidage de gaz, le flux d'air expiré est amené en totalité sur un catalyseur d'oxydation qui oxyde le monoxyde d'azote en dioxyde d'azote et ensuite, dans une unité de détection de gaz qui mesure la concentration de dioxyde d'azote, la concentration de monoxyde d'azote de l'air expiré pouvant être calculée à partir de la concentration de dioxyde d'azote.

16. Dispositif selon l'une quelconque des revendications 14 ou 15,
dans lequel la substance constituant le catalyseur d'oxydation est un composé appartenant au groupe formé par les sels permanganate ou perchlorate, déposé sur un support tel qu'une zéolithe, de l'oxyde d'aluminium ou du gel de silice.

17. Dispositif selon l'une quelconque des revendications précédentes,
dans lequel des signaux de détection traités dans un système d'évaluation électronique peuvent être transférés dans un réseau électronique de données.

18. Dispositif selon l'une quelconque des revendications 13 à 17,
dans lequel le dispositif de guidage de gaz est muni d'un dispositif d'étranglement fixe ou variable destiné à produire une résistance à l'écoulement du flux.

19. Procédé de fonctionnement d'un dispositif selon l'une quelconque des revendications 1 à 18, comprenant les étapes suivantes :
de l'air expiré est amené comme gaz à mesurer sur un catalyseur d'oxydation permettant d'oxyder NO en NO₂,
le gaz à mesurer est ensuite admis dans une unité de détection de gaz dans lequel la teneur en NO₂ est mesurée,
la teneur en NO est calculée à partir de la teneur en NO₂,
la régénération du système de détection de gaz est effectuée par balayage avec de l'air exempt de NOₓ.

20. Procédé selon la revendication 19,
dans lequel on prépare de l'air exempt de NOₓ destiné à être inspiré afin d'éliminer les influences atmosphériques.

21. Procédé selon la revendication 19 ou 20,
dans lequel de l'air exempt de NOₓ est obtenu par passage dans un épurateur ou dans un dispositif de séparation de NOₓ.

22. Utilisation d'un dispositif destiné à la détermination quantitative d'oxydes d'azote dans l'air expiré, selon l'une quelconque des revendications 1 à 18, pour déterminer la concentration du monoxyde d'azote dans l'air expiré.

23. Utilisation selon la revendication 22,
dans laquelle des signaux de mesure issus d'un système d'évaluation électronique du dispositif peuvent être transférés dans un système de réseau électronique.

24. Utilisation selon l'une des revendications 22 ou 23,
dans laquelle on utilise un dispositif de mesure destiné à la détection des maladies asthmatiques et des inflammations pulmonaires.
